# EUROPÄISCHE PATENTSCHRIFT

(11) **EP 3 169 689 B1**
(45) Veröffentlichungstag und Bekanntmachung des Hinweises auf die Patenterteilung: **05.09.2018**
(21) Anmeldenummer: 14742493.1
(22) Anmeldetag: 18.07.2014
(51) Int. Cl.: C07D 493/04

(54) **VERFAHREN ZUR HERSTELLUNG PRIMÄRER ISOHEXID-AMINE**
METHOD FOR THE SYNTHESIS OF PRIMARY ISOHEXIDE AMINES
PROCÉDÉ DE PRÉPARATION D'AMINES D'ISOHEXIDE PRIMAIRES

(43) Veröffentlichungstag der Anmeldung: 24.05.2017
(73) Patentinhaber: Rheinisch-Westfälische Technische Hochschule (RWTH) Aachen, 52056 Aachen (DE)
(72) Erfinder: ROSE, Marcus, 52074 Aachen (DE); PALKOVITS, Regina, 52074 Aachen (DE); ENGEL, Rebecca, Cardiff CF 24 3HB (GB); HAJI BEGLI, Alireza, 67305 Ramsen (DE); KRÖNER, Christine, 67283 Obrigheim/Pfalz (DE)
(74) Vertreter: Koster, Nico
(86) Internationale Anmeldenummer: PCT/EP2014/065556
(87) Internationale Veröffentlichungsnummer: WO 2016/008547

(56) Entgegenhaltungen:
- EP-A2- 0 312 253
- WO-A1-2007/107477
- WO-A1-2012/113475
- DE-A1-102011 004 465
- DE-A1-102011 075 162

## Beschreibung

Die Erfindung betrifft ein Verfahren zur Aminierung sekundärer Isohexid-Alkohole zu primären Aminen.

Biogene, d.h. aus erneuerbaren Rohstoffen herstellbare, Plattformchemikalien besitzen hohe wirtschaftliche Bedeutung, und ein weiteres signifikantes Wachstum des Anteils von Kunststoffen aus erneuerbaren Rohstoffen in der Zukunft ist angestrebt. Unter den wichtigsten biogenen Plattformchemikalien wird Sorbitol eine bedeutende Rolle beigemessen. Sorbitol wird als Bulkchemikalie hauptsächlich aus zucker- und stärkehaltigen Pflanzen hergestellt. Durch zweifache Dehydratisierung von Sorbitol, also Abspaltung von Wasser, kann Isosorbid gewonnen werden, ein Zuckeralkohol mit großem Potential als biogene Plattformchemikalie. Neben der Gewinnung von Isosorbid aus konventionell hergestelltem Sorbitol ist auch die Herstellung aus Cellulose direkt in einer einstufigen Reaktion möglich. Isosorbid ist das am besten zugängliche von drei Isohexiden. Isomannid kann auf analogem Weg zu Isosorbid ausgehend von Fructose über Mannitol hergestellt werden. Isoidid, dessen Vorläuferverbindung in der Natur nur in vernachlässigbar kleinen Mengen vorkommt, kann aus Isosorbid bzw. Isomannid durch eine einstufige Isomerisierungsreaktion gewonnen werden.

Isosorbid, Isomannid und Isoidid sowie deren verschiedene bifunktionelle Derivate besitzen hohes Potential für Anwendungen beispielsweise in biogenen Polymeren, Lösungsmitteln, Treibstoffzusätzen sowie in zahlreichen weiteren Bereichen. Neben dem Einsatz der biogenen Diole in Polymeren ist zunehmend die Verwendung multifunktioneller Amine, die aus biogenen Alkoholen hergestellt werden können, von Interesse, insbesondere die Verwendung biogener Amine für die Produktion von stickstoffhaltigen Kunststoffen wie Polyamiden und Polyurethanen. Insbesondere die Isohexide, bifunktionelle Zuckeralkohole, zeigen erstrebenswerte Eigenschaften als biogene Monomere in den entsprechenden Polymeren.

Die Herstellung von Isohexidaminen auf Basis von Dianhydrohexitolen ist prinzipiell bekannt. Bisher ist jedoch noch kein Syntheseverfahren zu deren Aminierung bekannt, das effizient genug für eine großtechnische Produktion der Isohexidamine, beispielsweise für den Bereich neuer biomasse-basierter Kunststoffe, ist. Entweder sind unkatalysierte mehrstufige Synthesen mit teilweise hoch reaktiven bzw. teilweise toxischen Zwischenstufen notwendig oder es werden im Labormaßstab Syntheserouten genutzt, die aufgrund der Bildung stöchiometrischer Mengen an Nebenprodukten für eine Aufskalierung nicht in Betracht gezogen werden können. Daher ist eine direkte katalytische Umwandlung der Dianhydrohexitole zu ihren Aminderivaten zu bevorzugen.
Weiterhin ist zwar eine homogen katalysierte Aminierung der Dianhydrohexitole bekannt, jedoch basieren diese auf molekularen Katalysatoren auf Basis teurer Edelmetallkomplexe mit anspruchsvollen und ebenfalls nur in Kleinstmengen verfügbaren zusätzlichen Liganden, beispielsweise molekulare Rutheniumkomplexe mit unterschiedlichen Phosphinliganden oder Pincer-Komplexe. Jedoch besitzen die homogenkatalysierten Reaktionen deutliche Nachteile. Diese Komplexe müssen aufwendig synthetisiert werden und sind daher teuer in der Herstellung. Sie sind meist empfindlich gegenüber Luft und Wasser, was die Verwendung in der Katalyse erschwert, da meist unter Inertgasatmosphäre und in wasserfreien organischen Lösungsmitteln gearbeitet werden muss. Weiterhin ist die Abtrennung des Katalysators vom Produkt aufwendig und energieintensiv. Insgesamt sind diese Reaktionsbedingungen für eine Aufskalierung und die Produktion technischer Mengen der Isohexid-Amine ungeeignet. Stand der Technik (homogene Katalysatore): DE 10 2011 004 465 Daher besteht ein Bedarf an alternativen Synthesemethoden der Isohexid-Amine. Der vorliegenden Erfindung lag daher die Aufgabe zu Grunde, ein Verfahren zur Verfügung zu stellen, dass mindestens einen der vorgenannten Nachteile des Standes der Technik überwindet. Insbesondere lag der vorliegenden Erfindung die Aufgabe zu Grunde ein Verfahren bereit zu stellen, das für die großtechnische Herstellung von biogenen Isohexid-Aminen verwendbar ist.

Diese Aufgabe wird gelöst durch ein Verfahren zur Herstellung eines primären Amins, umfassend die Schritte:
a) Bereitstellen wenigstens eines Dianhydrohexitols, und
b) Aminierung des Dianhydrohexitols durch Umsetzen mit Ammoniak,
wobei man die Aminierung mittels heterogener Katalyse unter Verwendung eines Hydrierkatalysators in Gegenwart von Wasserstoff durchführt.

Überraschend wurde gefunden, dass das erfindungsgemäße Verfahren die Umwandlung von Isohexiden zu primären Aminen und Aminoalkoholen unter Nutzung fester Katalysatoren in wässrigen Lösungen und unter milden Reaktionsbedingungen erlaubt. Geeignete feste Katalysatoren sind kommerziell erhältlich und ermöglichen die Reaktion unter vergleichsweise milden Reaktionsbedingungen in wässriger Lösung sowie in lösungsmittelfreien Systemen in der Substratschmelze. Die festen Katalysatoren können nach der Reaktion durch einfache Filtration abgetrennt und das Reaktionsgemisch weiterverarbeitet werden. Somit wird ein einschrittiges und wirtschaftliches Verfahren zur Verfügung gestellt, das heterogenkatalysiert in insbesondere vorteilhafter Weise in wässrigem Medium oder lösungsmittelfrei durchgeführt werden kann und selektiv in Bezug auf die Amine abläuft.

Unter Benutzung eines kommerziell verfügbaren heterogenen Katalysators, Ru/C, konnten milde Reaktionsbedingungen von 170 °C und 10 bar Wasserstoff zur Verfügung gestellt werden. Diese Parameter erlauben erstmals die Aufskalierung der Aminierung von Isohexiden in den technischen Maßstab sowie mit der dadurch verbundenen guten Verfügbarkeit der Amin-Monomere eine Weiterentwicklung und Kommerzialisierung von Produkten, insbesondere Kunststoffen, auf Basis biogener Amine. Die resultierenden Amine besitzen großes Marktpotential insbesondere in der Herstellung neuer Kunststoffe im Bereich der Polyamide sowie Polyurethane, sowie auch in anderen Marktsegmenten chemischer Endprodukte, wie Weichmachern, Detergenzien, Pharmazeutika und Agrochemikalien.

Unter dem Begriff "Aminierung" wird eine Reaktion verstanden, bei der eine oder mehrere Aminogruppen in ein Molekül eingeführt werden. Als Aminierungsreagenz ist Ammoniak verwendbar. Unter dem Begriff "heterogene Katalyse" wird eine Katalyse verstanden, bei der Katalysator und reagierende Verbindungen in unterschiedlichen Phasen vorliegen. Unter dem Begriff "Hydrierkatalysator" wird ein Katalysator verstanden, der eine Hydrierung katalysiert. Bei dem erfindungsgemäßen Verfahren liegt der heterogene Hydrierkatalysator als Feststoff vor, während das Substrat Dianhydrohexitol in Lösung oder einer lösungsmittelfreien Schmelze sowie die Reaktionspartner Ammoniak und Wasserstoff gasförmig und/oder in gelöster Form vorliegen.

Das in Schritt a) bereitgestellte Dianhydrohexitol ist vorzugsweise ausgewählt aus der Gruppe umfassend Isosorbid, Isomannid, Isoidid und/oder deren Mischungen. Dianhydrohexitole, auch "Isohexide" genannt, sind wie vorstehend beschrieben nach bekannten Verfahren synthetisierbar oder kommerziell erhältlich.

Die als "Isohexide" bezeichneten Dianhydrohexitole sind hetereocyclische Verbindungen, die durch zweifache Dehydratisierung von Hexitolen wie Sorbitol gewonnen werden können. Isosorbid besteht aus zwei ,V'-förmig kantenverknüpften Tetrahydrofuranringen mit zwei freien Hydroxygruppen. Die 2-O Hydroxygruppe ist exo-konfiguriert, während die 5-O-Hydroxygruppe endo-Konfiguration aufweist. Aufgrund der unterschiedlichen Konfiguration besitzen beide Gruppen eine unterschiedliche Reaktivität sowie eine sterisch unterschiedlich stark gehinderte Zugänglichkeit. So bildet die 5-O-*endo*-Hydroxygruppe eine intramolekulare Wasserstoffbrückenbindung zu dem gegenüberliegenden Ether-Sauerstoffatom, was die Nukleophilie der OH-Gruppe erhöht. Im Gegensatz ist die 2-O-*exo*-Hydroxygruppe sterisch ungehindert und tendiert zur Ausbildung intermolekularer Wasserstoffbrückenbindungen.

Neben Isosorbid existieren zwei Isomere: Isomannid, dessen beide Hydroxygruppen in *endo-*Konfiguration stehen, und Isoidid, dessen beide Hydroxylgruppen *exo*-Konfiguration aufweisen, die sich durch eine höhere Symmetrie auszeichnen, da beide Hydroxygruppen jeweils gleich konfiguriert vorliegen. Die Begriffe "Dianhydrohexitol" und "Isohexid" werden im Sinne der vorliegenden Erfindung synonym verwendet. Isosorbid wird auch als 1,4:3,6-Dianhydrosorbitol bzw. -glucitol, Isomannid als 1,4:3,6-Dianhydromannitol und Isoidid als 1,4:3,6-Dianhydroiditol bezeichnet. Die IUPAC-Bezeichnung für Isosorbid lautet (3*R*,3a*R*,6*S*,6a*R*)-Hexahydro-furo[3,2-*b*]furan-3,6-diol, für Isoidid (3*S*,3a*R*,6*S*,6a*R*)-Hexahydro-furo[3,2-b]furan-3,6-diol und für Isomannid (3*R*,3a*R*,6*R*,6a*R*)-Hexahydro-furo[3,2-b]furan-3,6-diol.

Das erfindungsgemäße Verfahren stellt ein heterogen katalysiertes Verfahren zur Aminierung von Isohexiden oder Dianhydrohexitolen zu Diamino-dianhydro-dideoxy-hexitolen und Monomamino-dianhydro-monodeoxy-hexitolen zur Verfügung. Bevorzugte heterogene Hydrierkatalysatoren basieren auf Metallen ausgewählt aus Ruthenium, Platin, Palladium und Nickel. Diese können auf einen Träger aufgebracht sein, insbesondere kohlenstoffhaltige Träger wie Kohle, Aktivkohle oder Ruß, oder Oxide wie Aluminiumoxid Al₂O₃. Hydrierkatalysatoren sind ebenfalls nicht-geträgert verwendbar, beispielsweise in Form von Nanopartikeln oder porösen Metallen wie Raney-Nickel. Die Katalysatoren können zudem in Form dotierter oder legierter Metalle aus mehreren Elementen vorliegen.

Bevorzugt verwendet man als Hydrierkatalysator einen geträgerten oder nicht geträgerten Metall-Katalysator eines oder mehrerer hydrieraktiver Übergangs- oder Edel-Metalle ausgewählt aus der Gruppe umfassend Platin, Palladium, Ruthenium, Iridium, Rhodium, Chrom, Molybdän, Wolfram, Vanadium, Nickel, Cobalt, Kupfer und/oder Eisen. Die Verwendung mehrerer Übergangs- oder Edel-Metalle schließt hierbei insbesondere hydrieraktive Verbindungen wie Kupferchromit und Zinkchromit ein. In bevorzugten Ausführungsformen verwendet man als Hydrierkatalysator einen geträgerten oder nicht geträgerten Metall-Katalysator eines Metalls ausgewählt aus der Gruppe umfassend Ruthenium, Platin, Palladium und/oder Nickel. Geträgerte Ruthenium-, Platin- und Palladium-Katalysatoren sind kommerziell erhältlich, beispielsweise Ru/C, Ru/Al₂O₃, Pd/C, Pt/C, ebenso wie Raney-Nickel. In vorteilhafter Weise zeigten die Metalle eine gute katalytische Aktivität für die Aminierungsreaktion der Isohexide. Insbesondere Platin und Ruthenium zeigten gute Aktivität. Vorzugsweise ist kohlenstoffgeträgertes Ruthenium, üblicherweise als Ru/C-Katalysator bezeichnet, verwendbar. Es konnte festgestellt werden, dass in Reaktionen, welche von Ru/C katalysiert wurden, in vorteilhafter Weise etwa 50 % Aminderivate entstanden. Ebenfalls bevorzugt verwendbar ist Ruthenium auf Al₂O₃ als Trägermaterial. Ru/Al₂O₃ zeigte ebenfalls eine gute Aktivität in der untersuchten Reaktion. Auch Raney-Nickel zeigte insbesondere bei höheren Temperaturen gute Ausbeuten. Demgegenüber wurde festgestellt, dass ohne Katalysator nur eine vernachlässigbare Menge der Isomere des Isohexid-Eduktes von jeweils unter einem halben Prozent gebildet wurde. Die Beladung der geträgerten Katalysatoren kann variieren und beispielsweise bei ca. 5 Gew% liegen. Insbesondere wurden gute Ergebnisse bei verwendeten Mengen von 2 mol% Ru/C, Ru/Al₂O₃, Pd/C oder Pt/C als katalytisch aktive Metallspezies in Bezug auf die Stoffmenge des jeweiligen Substrats erzielt. Für Raney-Nickel wurden gute Ergebnisse mit 20 mol% erzielt.

Es ist bevorzugt, dass der Schritt b) bei Überdruck, bezogen auf Atmosphärendruck, ausgeführt wird. Beispielsweise kann das Verfahren in einem Reaktor wie einem Druckautoklaven durchgeführt werden. In einem Druckautoklaven kann der Wasserstoff mit einem geeigneten Druck aufgepresst werden. In bevorzugten Ausführungsformen presst man den Wasserstoff mit einem Wasserstoffdruck im Bereich von ≥ 1 bar bis ≤ 25 bar, vorzugsweise im Bereich von ≥ 5 bar bis ≤ 25 bar, bevorzugt im Bereich von ≥ 10 bar bis ≤ 25 bar, in einem Reaktor auf. Hierbei entsprechen die Druckangaben jeweils dem aufgepressten Druck bei Raumtemperatur entsprechend 20±2°C. In vorteilhafter Weise sind bereits 5 bar Wasserstoffdruck ausreichend damit signifikante Mengen der Monoamine gebildet werden. Ein ebenfalls bevorzugter Wasserstoffdruck liegt im Bereich von ≥ 5 bar bis ≤ 15 bar, bevorzugt im Bereich von ≥ 10 bar bis ≤ 15 bar. In einem Bereich zwischen 10 und 25 bar wurde gefunden, dass die Produktzusammensetzung bezüglich der Amine recht konstant war. Es wird angenommen, dass Isomerisierungsreaktionen von höheren Wasserstoffdrücken profitieren können. Demgegenüber wurde festgestellt, dass die Aminierungsreaktion ohne Wasserstoff unter den verwendeten Bedingungen nicht ablief.

Von großem Vorteil ist, dass auf einen Ausschluss von Wasser und ein Arbeiten unter Schutzgas verzichtet werden kann, wodurch der Aufwand der Synthese deutlich reduziert und die Bedingung deutlich vereinfacht werden. Die festen Katalysatoren können nach der Reaktion leicht abgetrennt werden, beispielsweise durch einfache Filtration, und das Reaktionsgemisch weiterverarbeitet werden. In vorteilhafter Weise kann ein einfacher und im technischen Maßstab durchführbarer Zugang zu den Isohexid-Aminen mit guter Ausbeute und Selektivität zur Verfügung gestellt werden.

In bevorzugten Ausführungsformen führt man die Aminierung bei einer Temperatur im Bereich von ≥ 100°C bis ≤ 250°C, bevorzugt im Bereich von ≥ 120°C bis ≤ 230°C, vorzugsweise bei einer Temperatur im Bereich von ≥ 150°C bis ≤ 200°C, durch. Die Aminierung läuft bereits bei Temperaturen im Bereich von ≥ 100°C bis ≤ 150°C mit guter Ausbeute, jedoch kann eine Erhöhung der Temperatur zu einem schnelleren Ablauf der Reaktion sowie insbesondere einem größeren Anteil an Aminprodukten führen. Insbesondere eine Temperatur im Bereich von ≥ 150°C bis ≤ 200°C, beispielsweise von 170°C, kann zu über 80 % Anteil an Aminderivaten in der Produktlösung führen. Ebenfalls benötigt die Reaktion bei einer Temperatur im Bereich von ≥ 150°C bis ≤ 200°C nur wenige Stunden. Ab einer Temperatur von 250°C kann der Anteil an Nebenprodukten zunehmen.

Es wurde festgestellt, dass der Faktor Temperatur neben dem verwendeten Wasserstoffdruck und dem Verhältnis von Isohexid zu Ammoniak den größeren Einfluss auf die Aminierungsreaktion der Isohexide zeigte. Weitere Parameter, die untersucht wurden, waren die Reaktionszeit sowie die verwendete Rührgeschwindigkeit. Reaktionszeiten im Bereich von ≥ 24 h bis ≤ 48 h erwiesen sich als vorteilhaft, insbesondere in Bezug auf die Effizienz und Wirtschaftlichkeit der Reaktion. Es wurde festgestellt, dass eine Verdopplung der Reaktionszeit von 24 auf 48 Stunden zu einem größeren Anteil an Aminprodukten führte. Weiterhin erwiesen sich Rührgeschwindigkeiten im Bereich von ≥ 500 rpm bis ≤ 1000 rpm als vorteilhaft. Es wurde festgestellt, dass eine schnellere Rührgeschwindigkeit keinen Einfluss auf die Produktverteilung hatte.

Es ist für die großtechnische Herstellung von biogenen Aminen insbesondere vorteilhaft, dass das erfindungsgemäße Verfahren in wässrigem Medium oder lösungsmittelfrei in der Substratschmelze durchgeführt werden kann. Vorzugsweise ist Wasser als Lösungsmittel verwendbar. Dies kann den Vorteil zur Verfügung stellen, dass eine Aufarbeitung oder kostspielige Entsorgung organischer Lösungsmittel entfällt. Jedoch können organische Lösungsmittel ebenfalls geeignet sein, beispielsweise *tert*-Amylalkohol. Es ist jedoch ein großer Vorteil, dass auf organische Lösungsmittel verzichtet werden kann. Zudem wurde festgestellt, dass die Verwendung eines organischen Lösungsmittels keinen Einfluss auf die Produktverteilung hatte.

In bevorzugten Ausführungsformen führt man die Aminierung in wässriger Lösung oder Lösungsmittel-frei durch. Insbesondere konnte gezeigt werden, dass die Aminierung der Isohexide auch lösungsmittelfrei möglich ist. Lösungsmittel-frei kann die Aminierung in einer Schmelze des Dianhydrohexitols durchgeführt werden. Die Isohexide schmelzen bei einer Temperatur im Bereich von 60°C bis 70°C und liegen bei den bevorzugten Reaktionstemperaturen somit als Schmelze vor. Ammoniak kann zu einer Schmelze des Eduktes beispielsweise in flüssiger Form eingebracht werden.

Eine Durchführung der Aminierung in wässriger Lösung oder Lösungsmittel-frei erlaubt verschiedene Möglichkeiten des Vorlegens des Isohexids und der Zugabe von Ammoniak. In bevorzugten Ausführungsformen wird in Schritt a) das Dianhydrohexitol festförmig oder in wässriger Lösung vorgelegt. In weiter bevorzugten Ausführungsformen wird Ammoniak gasförmig oder flüssigförmig, oder als wässrige Ammoniumlösung zugesetzt. In vorteilhafter Weise sind Kombinationen hiervon möglich. Ammoniak kann gasförmig oder in flüssiger Form, beispielsweise mit Hilfe einer Dosierpumpe in einen Autoklaven, eingebracht werden.

Beispielsweise kann das Dianhydrohexitol festförmig vorgelegt werden und Ammoniak gasförmig oder in flüssiger Form zugegeben werden. Es ist ebenfalls bevorzugt, eine wässrige Ammoniaklösung zu verwenden. In dieser kann das Isohexid gelöst werden. Es ist insbesondere bevorzugt, die Aminierung des Isohexids in wässriger Ammoniaklösung durchzuführen, da diese nicht nur als Ammoniakquelle, sondern auch als Lösungsmittel dienen kann. Das Isohexid kann auch in Wasser gelöst werden, und der Ammoniak gasförmig oder flüssigförmig, oder ebenfalls in Form einer wässrigen Lösung zugegeben werden. Beispielsweise kann Wasser als Lösungsmittel für das Isohexid verwendet und gas- oder flüssigförmiger Ammoniak zugesetzt werden. Es wurde festgestellt, dass Versuche, in denen in Wasser gearbeitet wurde, und Ammoniak separat zugegeben wurde, nur geringe Unterschiede in der Produktverteilung zu Versuchen aufwiesen, in denen das Isohexid in einer wässrigen Ammoniaklösung vorgelegt wurde.

Verwendbare wässrige Ammoniaklösungen können variable Konzentrationen an Ammoniak aufweisen, beispielsweise im Bereich von ≥ 10 Gew.-% bis ≤ 50 Gew.-%, vorzugsweise im Bereich von ≥ 20 Gew.-% bis ≤ 30 Gew.-%, Ammoniak bezogen auf das Gesamtgewicht der Lösung. Insbesondere hat sich eine 25 %ige (m/m) Lösung als vorteilhaft erwiesen.

Es ist ebenfalls möglich Ammoniumsalze, die unter den erfindungsgemäßen Reaktionsbedingungen durch Zersetzung Ammoniak freisetzen, in einem Lösungsmittel zu verwenden. Bevorzugt sind anorganische Ammoniumsalze wie Ammoniumcarbonat, Ammoniumsulfat, Ammoniumchlorid oder Ammoniumnitrat.

Ammoniak kann bezogen auf die Hydroxygruppen des Dianhydrohexitols in stöchiometrischen Mengen oder im Überschuss verwendet werden. Ammoniak kann im Bereich von ≥ 2 Äquivalenten (eq.) bis ≤ 20 Äquivalenten (eq.), bezogen auf das Isohexid, vorliegen. In bevorzugten Ausführungsformen liegt in Schritt b) Ammoniak im Bereich von ≥ 5 eq. bis ≤ 20 eq., vorzugsweise im Bereich von ≥ 10 eq. bis ≤ 16 eq., bevorzugt im Bereich von ≥ 11 eq. bis ≤ 13 eq., bezogen auf das Isohexid, vor. Bei einem Überschuss größer als vier Äquivalente konnten bessere Ausbeuten an Aminderivaten, insbesondere Diaminen, erzielt werden. Durch einen größeren Überschuss an Ammoniak, beispielsweise von 27 Äquivalenten, wurde die Produktverteilung nicht in Richtung einer größeren Ausbeute an Aminen beeinflusst. Insbesondere im Bereich von ≥ 10 eq. bis ≤ 16 eq., bevorzugt im Bereich von ≥ 11 eq. bis ≤ 13 eq., bezogen auf das Isohexid, können gute Ergebnisse zur Verfügung gestellt werden. 11 Äquivalenten (eq.) Ammoniak bezüglich des Isosorbids entsprechen hierbei etwa 5 Äquivalenten bezogen auf eine Hydroxylgruppe.

Es wird angenommen, dass die Herstellung von Isohexid-Aminen in zwei Reaktionsschritten verläuft, wobei zunächst ein Aminoalkohol, auch Monoamin genannt, gebildet wird, welcher anschließend zum Diamin weiterreagieren kann. Es wird weiter angenommen, dass die katalytische Aminierung von Dianhydrohexitolen in drei Schritten verläuft, wobei nach einer metallkatalysierten Dehydrierung des Alkohols zum Keton die Iminbildung erfolgt, welche unkatalysiert abläuft, und anschließend das Imin katalytisch zum Diamin-Produkt hydriert wird. Das Imin bildet ein prochirales Zentrum. Daher können beide möglichen Konfigurationen der Aminfunktion (*endo*/*exo*) entstehen, unabhängig davon welche Konfiguration die korrespondierende Hydroxylgruppe vorher hatte. Aus diesem Grund und der Tatsache, dass sowohl doppelt als auch einfachsubstituierte Aminderivate entstehen können, kann das Produktspektrum vier Aminoalkohole, auch Monoamine genannt, und drei Diamine mit unterschiedlicher Stereochemie enthalten. Außerdem besitzt auch das Keton als Zwischenstufe ein prochirales Zentrum und kann ebenfalls wieder hydriert werden, sodass, unabhängig vom Startmaterial, alle drei Isohexidisomere gebildet werden können.

Bei einem geringen Überschuss an Ammoniak von 4 eq. konnte eine Ausbeute ausschließlich an Monoaminen festgestellt werden, während bei einem Überschuss von 11 eq. bessere Ausbeuten an Aminderivaten, insbesondere Diaminen, festgestellt wurden.

Weiterhin kann die Produktverteilung substratabhängig durch Auswahl des Isohexid-Eduktes beeinflusst werden. Das in Schritt a) bereitgestellte Dianhydrohexitol ist vorzugsweise ausgewählt aus der Gruppe umfassend Isosorbid, Isomannid, Isoidid und/oder deren Mischungen. Es konnte festgestellt werden, dass ausgehend von Isomannid als Startmaterial etwas mehr Aminderivate als bei der Verwendung von Isosorbid gebildet wurden. Weiterhin wurde ausgehend von Isomannid eine deutlich größere Menge an Diaminen gebildet.

Insgesamt kann ein Verfahren zur Verfügung gestellt werden, das unter Verwendung kommerziell verfügbarer heterogener Katalysatoren und industriell skalierbaren Bedingungen eine Aminierung der Isohexide erlaubt. Insbesondere bevorzugt ist Ru/C, bevorzugte milde Reaktionsbedingungen sind 170 °C und 10 bar Wasserstoffdruck. Weiterhin haben sich ein Arbeiten in wässriger Ammoniaklösung und ein Überschuss von 11 eq. bezogen auf das Isohexid als ganz besonders vorteilhaft erwiesen. Hierbei kann die Reaktion innerhalb von 24 Stunden mit guter Ausbeute geführt werden. Diese Parameter erlauben erstmals die Aufskalierung dieser Reaktion in den technischen Maßstab sowie mit der dadurch verbundenen guten Verfügbarkeit der Amin-Monomere eine Weiterentwicklung und Kommerzialisierung der Produkte.

Beispiele und Figuren, die der Veranschaulichung der vorliegenden Erfindung dienen, sind nachstehend angegeben.

Hierbei zeigt:

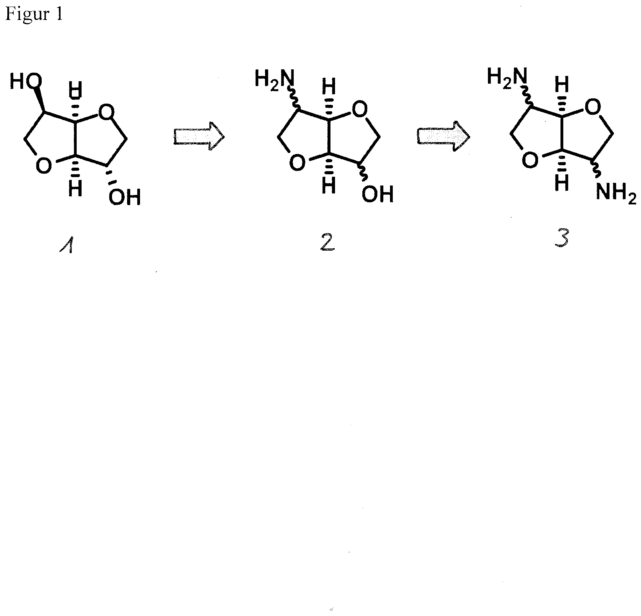

- Figur 1: die Herstellung von Isohexid-Aminen in zwei Reaktionsschritten am Beispiel von Isosorbid.

Die Figur 1 zeigt, dass durch Umsetzen mit NH₃ aus Isosorbid oder 1,4:3,6-Dianhydrosorbitol 1 zunächst der Aminoalkohol 2, auch Monoamin genannt, gebildet wird, welcher anschließend ebenfalls durch Reaktion mit NH₃ zum Diamin 3 weiterreagiert.
Erfindungsgemäß erfolgt die Aminierung des Isosorbids 1 mittels heterogener Katalyse unter Verwendung eines Hydrierkatalysators in Gegenwart von Wasserstoff.

Allgemeine Arbeitsvorschrift für die Aminierung:
Die Versuche wurden in einem 45 mL Druckautoklaven durchgeführt. Die Versuche in 5 mL organischem Lösemittel oder Wasser wurden mit 1 g Isohexid (6,8 mmol) und 2 mol% Katalysator (0,137 mmol Metallspezies) in Bezug auf die katalytisch aktive Metallspezies durchgeführt. Eine Ausnahme bildeten die Versuche mit Raney-Nickel, bei denen mit 20 mol% gearbeitet wurde. In den Versuchen ohne Lösemittel wurden 5 g Isohexid (34,2 mmol) eingesetzt und die Katalysatormenge entsprechend angepasst. Der Ammoniak wurde entweder pur und in flüssiger Form mit Hilfe einer Dosierpumpe in den Autoklaven eingebracht oder es wurde eine 25 %ige (m/m) wässrige Ammoniaklösung genutzt, welche zeitgleich als Lösungsmittel diente.

Bei jedem Versuch wurde der Autoklav nach Aufpressen des Wasserstoffdrucks, wobei die Druckangaben jeweils dem aufgepressten Druck bei Raumtemperatur (20±2°C) entsprachen, auf die entsprechende Temperatur erhitzt und die Reaktion nach 24 bis 48 h inklusive der Aufheizphase gestoppt. Der Katalysator wurde von der Reaktionslösung abfiltriert und die Proben nach einer Derivatisierung mittels Gas-Chromatographie (GC) untersucht.

Gas-Chromatographie:
Die Analyse der Produktlösungen erfolgte über Gas-Chromatographie. Hierfür wurden die Proben zunächst mittels Chlortrimethylsilan derivatisiert. Mittels Gas-Chromatographie wurden die einzelnen Produkte aufgetrennt. Ferner wurden die Mono- und die Diamine mit Hilfe von Gaschromatographie mit Massenspektrometrie-Kopplung (GC-MS) unterschieden. Zur Auswertung wurden die Flächen der einzelnen Komponenten ermittelt und auf 100 % normiert.

### Beispiel 1

### Aminierung von Isosorbid mit verschiedenen Katalysatoren

Die Versuche zur Aminierung von Isosorbid mit verschiedenen Katalysatoren wurden wie in der allgemeinen Versuchsvorschrift beschrieben durchgeführt, wobei 5 mL einer 25 %igen (m/m) wässrigen Ammoniaklösung verwendet wurden. Diese diente als Ammoniakquelle sowie als Lösungsmittel für 1 g Isosorbid (6,8 mmol, Alfa Aesar, 98%). Die verwendeten 5 mL der 25 %ige Lösung entsprachen 11 Äquivalenten (eq.) Ammoniak bezüglich des Isosorbids bzw. 5 bezogen auf eine Hydroxylgruppe. Hierzu wurden jeweils 277 mg Ru/C (Sigma-Aldrich), 277 mg Ru/Al₂O₃, 292 mg Pd/C (, 535 mg Pt/C und 180 mg eines Slurries von Raney-Nickel gegeben. Dies entsprach jeweils 2 mol% Katalysator (0,137 mmol) in Bezug auf die katalytisch aktive Metallspezies der geträgerten Katalysatoren. Die Beladung der geträgerten Katalysatoren betrug jeweils 5 Gew%. Bei Versuchen mit Raney-Nickel wurde mit 20 mol% gearbeitet. Als Kontrolle wurden Blindversuche ohne Katalysator durchgeführt. Die Versuche wurden bei einer Temperatur von 170°C, 11 eq. NH₃, 10 bar Wasserstoffdruck, 500 rpm Rührgeschwindigkeit und 24 Stunden Reaktionszeit durchgeführt.

Es zeigte sich, dass Platin und Ruthenium moderate bis gute Aktivität zeigen. In der Reaktion, welche von Ru/C katalysiert wurde, entstanden etwa 50 % Aminderivate. Es wurden für Platin und Ruthenium jeweils 1 % bzw. 1 % eines Gemisches der Diamine Diaminoisosorbid, Diaminoisomannid und Diaminoisoidid erhalten und 18 % bzw. 49 % der vier Monoamine. Pd/C zeigte eine etwas geringere Aktivität in der Aminierungsreaktion und auch die Aktivität von Raney-Nickel war im Vergleich geringer. Raney-Nickel katalysiert hauptsächlich die Isomerisierung des Zuckeralkohols. In dem Vergleichsversuch ohne Katalysator wurde nur eine vernachlässigbare Menge der Isomeren von Isosorbid von jeweils unter einem halben Prozent erhalten.

### Beispiel 2

### Aminierung von Isosorbid unter Variation des Wasserstoffdrucks

Zur Untersuchung des Einflusses des Wasserstoffdrucks wurden Versuche mit 5 bar, 10 bar, 15 bar und 25 bar sowie ohne Wasserstoff durchgeführt. Die Versuche wurden wie in der allgemeinen Versuchsvorschrift beschrieben durchgeführt, wobei 5 mL einer 25 %igen (m/m) wässrigen Ammoniaklösung als Ammoniakquelle sowie als Lösungsmittel für 1 g Isosorbid (6,8 mmol) verwendet wurden. Hierzu wurden 2 mol% Ru/C als Katalysator (0,137 mmol) in Bezug auf die katalytisch aktive Metallspezies gegeben. Die Versuche wurden mit Ru/C als Katalysator, 11 eq. NH₃, bei einer Temperatur von 170°C, 500 rpm Rührgeschwindigkeit, 24 Stunden Reaktionszeit sowie einem Wasserstoffdruck von jeweils 5 bar, 10 bar, 15 bar oder 25 bar sowie ohne Wasserstoff durchgeführt.

Es zeigte sich, dass die Reaktion unter den genutzten Bedingungen ohne Wasserstoff nicht ablief. 5 bar Wasserstoffdruck erwiesen sich bereits als ausreichend damit signifikante Mengen von 44 % der Monoamine gebildet wurden. Zwischen den Ergebnissen der Versuche mit 10, 15 und 25 bar waren keine deutlichen Unterschiede in der Produktzusammensetzung bezüglich der Amine zu erkennen. Es wurden jeweils 50 %, 49 % bzw. 47 % eines Gemisches der Monoamine, sowie jeweils < 3 % eines Gemisches der Diamine erhalten.

Weiterhin wurde festgestellt, dass mit steigendem Druck größere Mengen an Isoidid gebildet wurden. Es wird angenommen, dass die Isomerisierungsreaktion zu Isoidid von höheren Wasserstoffdrücken profitieren.

### Beispiel 3

### Aminierung von Isosorbid unter Variation verschiedener Parameter

Die Versuche wurden wie in der allgemeinen Versuchsvorschrift beschrieben durchgeführt. Ein Versuchsansatz unter Verwendung von 5 mL 25 %iger (m/m) wässriger Ammoniaklösung entsprechend 11 eq. NH₃, 1 g Isosorbid (6,8 mmol), 2 mol% Ru/C als Katalysator (0,137 mmol), einer Temperatur von 170°C, 500 rpm Rührgeschwindigkeit, 24 Stunden Reaktionszeit sowie einem Wasserstoffdruck von 10 bar wurde als Standard gesetzt. In fünf Versuchen wurde hiervon abweichen jeweils ein Parameter verändert, wobei eine Temperatur von 200°C, eine Reaktionszeit von 48 Stunden, eine Rührgeschwindigkeit von 1000 rpm, die Verwendung von 2 mol% Ru/Al₂O₃ als Katalysator sowie von 16 Äquivalenten NH₃ untersucht wurden.

Es zeigte sich, dass ein etwas größerer Überschuss von 16 eq. an Ammoniak sowie eine schnellere Rührgeschwindigkeit keinen Einfluss auf die Produktverteilung hatten. Eine Verdopplung der Reaktionszeit führt ebenso zu einem größeren Anteil an Aminprodukten wie eine Erhöhung der Temperatur. Letzteres führt sogar zu über 80 % Anteil an Aminderivaten in der Produktlösung. Es wurden 79% Monoamine und 3,4% Diamine erhalten. Weiterhin zeigte sich, dass Al₂O₃ als Trägermaterial für Ruthenium ebenfalls gute Ergebnisse zeigte, so zeigte Ru/Al₂O₃ ebenfalls eine gute Aktivität in der untersuchten Reaktion.

### Beispiel 4

### Aminierung von Isomannid

Die Aminierung von Isomannid wurde wie in der allgemeinen Versuchsvorschrift beschrieben durchgeführt, wobei 1 g Isommanid (6,8 mmol, Sigma Aldrich, 95%) als Edukt in 5 mL 25 %iger (m/m) wässrigen Ammoniaklösung entsprechend 11 eq. NH₃, mit 2 mol% Ru/C als Katalysator (0,137 mmol), bei einer Temperatur von 170°C, 500 rpm Rührgeschwindigkeit, 24 Stunden Reaktionszeit und einem Wasserstoffdruck von 10 bar eingesetzt wurde. Zum Vergleich wurde unter ansonsten identischen Bedingungen 1 g Isosorbid als Edukt umgesetzt.

Ausgehend von Isomannid als Startmaterial wurden im Vergleich zu Isosorbid etwas mehr Aminderivate gebildet, 59 % im Vergleich zu 50 %. Zudem wurden bevorzugt zwei andere Monoamine gebildet. Ausgehend von Isomannid wurden mit einem Anteil an 16 % des Produktgemisches deutlich mehr Diamine erhalten als < 1 % bei Verwendung von Isosorbid.

Weiterhin wurde festgestellt, dass ausgehend von Isomannid die Monoamine mit einer Stereochemie gebildet wurden, welche in der Reaktion ausgehend von Isosorbid nicht entstanden. Es wird vermutet, dass dies auf die unterschiedliche Konfiguration bzw. Reaktivität der Hydroxylgruppen zurückgeführt werden kann. Die *endo*-konfigurierte Hydroxylgruppe scheint bevorzugt dehydriert zu werden und dementsprechend auch aminiert. Da im Isomannid beide Hydroxylgruppen eine *endo*-Konfiguration aufweisen, sollte die verbliebene Hydroxylgruppe in beiden Aminoalkoholen auch *endo*-konfiguriert sein. Im Isosorbid gibt es sowohl eine *endo-* als auch eine exo-konfigurierte Hydroxylgruppe und aus den genannten Gründen sollte demnach die verbliebene Hydroxylgruppe in den Aminoalkoholen aus Isosorbid *exo*-konfiguriert sein. Es wird vermutet, dass daher ausgehend von Isomannid eine deutlich größere Menge an Diaminen gebildet wird.

### Beispiel 5

### Aminierung von Isomannid unter Variation verschiedener Parameter

Die Versuche wurden wie in der allgemeinen Versuchsvorschrift beschrieben durchgeführt. Ein Versuchsansatz unter Verwendung von 5 mL 25 %iger (m/m) wässrigen Ammoniaklösung entsprechend 11 eq. NH₃, 1 g Isomannid (6,8 mmol), 2 mol% Ru/C als Katalysator (0,137 mmol), einer Temperatur von 170°C, 500 rpm Rührgeschwindigkeit, 24 Stunden Reaktionszeit sowie einem Wasserstoffdruck von 10 bar wurde als Standard gesetzt. In sechs Versuchen wurde hiervon abweichen jeweils ein Parameter verändert. Zum einen wurden in zwei Ansätzen eine Temperatur von 200°C sowie eine Reaktionszeit von 48 Stunden untersucht.

Weiterhin wurden Versuche in Wasser oder *tert*-Amylalkohol als Lösungsmittel sowie lösungsmittelfreie Versuche durchgeführt. Dafür wurde reiner, flüssiger Ammoniak in den Autoklaven dosiert. Es wurde in zwei Ansätzen 1 g Isomannid in 5 mL Wasser vorgelegt und Ammoniak im Verhältnis von 13 oder 27 Äquivalenten in flüssiger Form mit Hilfe einer Dosierpumpe in den Autoklaven eingebracht. In einem weiteren Ansatz wurde 1 g Isomannid in 5 mL *tert*.-Amylalkohol vorgelegt und Ammoniak im Verhältnis von 13 Äquivalenten in flüssiger Form in den Autoklaven eingebracht. In einem Ansatz ohne Lösemittel wurden 5 g Isomannid (34,2 mmol) eingesetzt und die Katalysatormenge entsprechend angepasst. Der Ammoniak wurde in einem Verhältnis von 4 Äquivalenten ebenfalls in flüssiger Form mit Hilfe einer Dosierpumpe in den Autoklaven eingebracht.

Es wurde festgestellt, dass auch bei der Verwendung von Isomannid als Edukt mit längerer Reaktionszeit und höherer Temperatur die Anteile an Aminderivaten zunahmen. So wurden im Ansatz bei einer Temperatur von 170°C und 24 Stunden Reaktionszeit 59 % Aminderivate erhalten, während bei 200°C 90 % und bei einer Reaktionszeit von 48 Stunden 71 % erhalten wurden. Wie in den Reaktionen mit Isosorbid zeigte der Faktor Temperatur den größeren Einfluss.

Die Versuche, in denen in Wasser als Lösungsmittel gearbeitet und Ammoniak separat zugegeben wurde, wiesen nur geringe Unterschiede auf zu der Verwendung von wässriger Ammoniaklösung auf. Bei einem ähnlichen Ammoniaküberschuss von 11 zu 13 eq waren in letzteren die Anteile an Aminderivaten etwas geringer. Ebenfalls wurde festgestellt, dass zwei der Monoamine nicht gebildet wurden. Auch ein größerer Überschuss an Ammoniak von 27 eq. wies gegenüber 11 eq. keinen merklichen Einfluss auf die Produktverteilung auf. Ebenfalls wurde festgestellt, dass ein Wechsel zu einem organischen Lösungsmittel, *tert-*Amylalkohol, keinen merklichen Einfluss auf die Produktverteilung aufwies.

Weiterhin zeigte die Aminierung von Isomannid ohne Lösemittel ebenfalls eine Bildung von Aminderivaten. Dies zeigt, dass die Aminierung von Isomannid auch lösungsmittelfrei möglich ist. Da nur ein geringer Überschuss an Ammoniak, welcher in diesem Versuch mit etwa 4 eq. deutlich unter den als Vergleich verwendeten 11 eq. lag, resultierte eine geringere Ausbeuten an Aminderivaten, welche ausschließlich Monoamine waren.

Insgesamt zeigen die Beispiele, dass die Umsetzung der biogenen Alkohole Isosorbid und Isomannid zu ihren Aminderivaten mittels heterogener Katalyse unter Verwendung eines Hydrierkatalysators in Gegenwart von Wasserstoff in wässriger Lösung und sogar lösungsmittelfrei erzielt werden konnte. Unter Verwendung eines kommerziell verfügbaren heterogenen Katalysators, Ru/C, konnten milde Reaktionsbedingungen von 170 °C und 10 bar Wasserstoff erzielt werden, die erstmals die Aufskalierung dieser Reaktion in den technischen Maßstab erlauben.

## Patentansprüche

1. Verfahren zur Herstellung eines primären Amins, umfassend die Schritte:
a) Bereitstellen wenigstens eines Dianhydrohexitols, und
b) Aminierung des Dianhydrohexitols durch Umsetzen mit Ammoniak,
**dadurch gekennzeichnet, dass** man die Aminierung mittels heterogener Katalyse unter Verwendung eines Hydrierkatalysators in Gegenwart von Wasserstoff durchführt.

2. Verfahren nach Anspruch 1, **dadurch gekennzeichnet, dass** als Hydrierkatalysator ein geträgerter oder nicht geträgerter Metall-Katalysator eines oder mehrerer hydrieraktiver Übergangs- oder Edel-Metalle ausgewählt aus der Gruppe umfassend Platin, Palladium, Ruthenium, Iridium, Rhodium, Chrom, Molybdän, Wolfram, Vanadium, Nickel, Cobalt, Kupfer und/oder Eisen, insbesondere ausgewählt aus der Gruppe umfassend Ruthenium, Platin, Palladium und/oder Nickel, vorzugsweise ein Ru/C-Katalysator, verwendet wird.

3. Verfahren nach Anspruch 1 oder 2, **dadurch gekennzeichnet, dass** man den Wasserstoff mit einem Wasserstoffdruck im Bereich von ≥ 1 bar bis ≤ 25 bar, vorzugsweise im Bereich von ≥ 5 bar bis ≤ 25 bar, bevorzugt im Bereich von ≥ 10 bar bis ≤ 25 bar, in einen Reaktor aufpresst.

4. Verfahren nach einem der vorherigen Ansprüche, **dadurch gekennzeichnet, dass** man die Aminierung bei einer Temperatur im Bereich von ≥ 100°C bis ≤ 250°C, bevorzugt im Bereich von ≥ 120°C bis ≤ 230°C, vorzugsweise bei einer Temperatur im Bereich von ≥ 150°C bis ≤ 200°C, durchführt.

5. Verfahren nach einem der vorherigen Ansprüche, **dadurch gekennzeichnet, dass** man die Aminierung in wässriger Lösung oder Lösungsmittel-frei durchführt.

6. Verfahren nach einem der vorherigen Ansprüche, **dadurch gekennzeichnet, dass** in Schritt a) das Dianhydrohexitol festförmig oder in wässriger Lösung vorgelegt wird.

7. Verfahren nach einem der vorherigen Ansprüche, **dadurch gekennzeichnet, dass** Ammoniak gasförmig oder flüssigförmig, oder als wässrige Ammoniumlösung zugesetzt wird.

8. Verfahren nach einem der vorherigen Ansprüche, **dadurch gekennzeichnet, dass** in Schritt b) Ammoniak im Bereich von ≥ 5 eq. bis ≤ 20 eq., vorzugsweise im Bereich von ≥ 10 eq. bis ≤ 16 eq., bevorzugt im Bereich von ≥ 11 eq. bis ≤ 13 eq., bezogen auf das Dianhydrohexitol, vorliegt.

9. Verfahren nach einem der vorherigen Ansprüche, **dadurch gekennzeichnet, dass** das in Schritt a) bereitgestellte Dianhydrohexitol ausgewählt ist aus der Gruppe umfassend Isosorbid, Isomannid, Isoidid und/oder deren Mischungen.

## Claims

1. Method for preparing a primary amine comprising the steps of:
a) providing at least one dianhydrohexitol, and
b) amination of the dianhydrohexitol by reaction with ammonia,
**characterized in that** the amination is carried out by heterogeneous catalysis using a hydrogenation catalyst in the presence of hydrogen.

2. Method according to Claim 1, **characterized in that** the hydrogenation catalyst used is a supported or unsupported metal catalyst of one or more hydrogenation-active transition metals or noble metals selected from the group comprising platinum, palladium, ruthenium, iridium, rhodium, chromium, molybdenum, tungsten, vanadium, nickel, cobalt, copper and/or iron, especially selected from the group comprising ruthenium, platinum, palladium and/or nickel, preferably a Ru/C catalyst.

3. Method according to Claim 1 or 2, **characterized in that** the hydrogen is pressurized in a reactor to a hydrogen pressure in the range from ≥ 1 bar to ≤ 25 bar, preferably in the range from ≥ 5 bar to ≤ 25 bar, preferably in the range from ≥ 10 bar to ≤ 25 bar.

4. Method according to any of the preceding claims, **characterized in that** the amination is carried out at a temperature in the range from ≥ 100°C to ≤ 250°C, preferably in the range from ≥ 120°C to ≤ 230°C, preferably at a temperature in the range from ≥ 150°C to ≤ 200°C.

5. Method according to any of the preceding claims, **characterized in that** the amination is carried out in aqueous solution or solvent-free.

6. Method according to any of the preceding claims, **characterized in that** the dianhydrohexitol in step a) is initially charged in solid form or in aqueous solution.

7. Method according to any of the preceding claims, **characterized in that** ammonia is added in the form of a gas or liquid or as an aqueous ammonium solution.

8. Method according to any of the preceding claims, **characterized in that** the ammonia in step b) is present in the range from ≥ 5 eq. to ≤ 20 eq., preferably in the range from ≥ 10 eq. to ≤ 16 eq., preferably in the range from ≥ 11 eq. to ≤ 13 eq., based on the dianhydrohexitol.

9. Method according to any of the preceding claims, **characterized in that** the dianhydrohexitol provided in step a) is selected from the group comprising isosorbide, isomannide, isoidide and/or mixtures thereof.

## Revendications

1. Procédé de fabrication d'une amine primaire, comprenant les étapes suivantes :
a) la préparation d'au moins un dianhydrohexitol et
b) l'amination du dianhydrohexitol par mise en réaction avec de l'ammoniac,
**caractérisé en ce que** l'amination est réalisée par catalyse hétérogène en utilisant un catalyseur d'hydrogénation en présence d'hydrogène.

2. Procédé selon la revendication 1, **caractérisé en ce qu'**un catalyseur métallique supporté ou non supporté d'un ou de plusieurs métaux de transition ou nobles actifs pour l'hydrogénation choisis dans le groupe comprenant le platine, le palladium, le ruthénium, l'iridium, le rhodium, le chrome, le molybdène, le tungstène, le vanadium, le nickel, le cobalt, le cuivre et/ou le fer, notamment choisis dans le groupe comprenant le ruthénium, le platine, le palladium et/ou le nickel, de préférence un catalyseur Ru/C, est utilisé en tant que catalyseur d'hydrogénation.

3. Procédé selon la revendication 1 ou 2, **caractérisé en ce que** l'hydrogène est injecté dans un réacteur à une pression d'hydrogène dans la plage allant de ≥ 1 bar à ≤ 25 bar, de préférence dans la plage allant de ≥ 5 bar à ≤ 25 bar, avantageusement dans la plage allant de ≥ 10 bar à ≤ 25 bar.

4. Procédé selon l'une quelconque des revendications précédentes, **caractérisé en ce que** l'amination est réalisée à une température dans la plage allant de ≥ 100 °C à ≤ 250 °C, de préférence dans la plage allant de ≥ 120 °C à ≤ 230 °C, avantageusement à une température dans la plage allant de ≥ 150 °C à ≤ 200 °C.

5. Procédé selon l'une quelconque des revendications précédentes, **caractérisé en ce que** l'amination est réalisée dans une solution aqueuse ou sans solvant.

6. Procédé selon l'une quelconque des revendications précédentes, **caractérisé en ce qu'**à l'étape a), le dianhydrohexitol est chargé sous forme solide ou dans une solution aqueuse.

7. Procédé selon l'une quelconque des revendications précédentes, **caractérisé en ce que** l'ammoniac est ajouté sous forme gazeuse ou sous forme liquide, ou sous la forme d'une solution aqueuse d'ammonium.

8. Procédé selon l'une quelconque des revendications précédentes, **caractérisé en ce qu'**à l'étape b), l'ammoniac est présent dans la plage allant de ≥ 5 éq. à ≤ 20 éq., de préférence dans la plage allant de ≥ 10 éq. à ≤ 16 éq., avantageusement dans la plage allant de ≥ 11 éq. à ≤ 13 éq., par rapport au dianhydrohexitol.

9. Procédé selon l'une quelconque des revendications précédentes, **caractérisé en ce que** le dianhydrohexitol préparé à l'étape a) est choisi dans le groupe comprenant l'isosorbide, l'isomannide, l'isoidide et/ou leurs mélanges.
